(19) Europäisches Patentamt

European Patent Office

Office européen des brevets

(11) EP 0 864 319 A1

## (12) DEMANDE DE BREVET EUROPEEN

(43) Date de publication:
16.09.1998 Bulletin 1998/38

(51) Int Cl.$^6$: **A61K 7/48**, A61K 7/06,
A61K 7/32, A61K 7/02

(21) Numéro de dépôt: 98400398.8

(22) Date de dépôt: 19.02.1998

(84) Etats contractants désignés:
AT BE CH DE DK ES FI FR GB GR IE IT LI LU MC
NL PT SE

(30) Priorité: 14.03.1997 FR 9703117

(71) Demandeur: L'OREAL
75008 Paris (FR)

(72) Inventeurs:
• Dupuis, Christine
75018 Paris (FR)

• Samain, Henri
91570 Bievres (FR)

(74) Mandataire: Miszputen, Laurent
L'OREAL
Département Propriété Industrielle
Centre Charles Zviak
90, rue du Général Roguet
92583 Clichy Cédex (FR)

(54) **Composition topique comprenant un gélifiant hydrophile de type polyester sulfoné**

(57) La présente invention concerne une composition topique comprenant une phase aqueuse laquelle comprend un agent gélifiant hydrophile, le gélifiant hydrophile étant un oligomère copolyester téréphtalique hydrosoluble ou hydrodispersable comprenant essentiellement des motifs répétitifs dicarboxylates de formule (I):

$$[-CO-A-CO-O-(CH2-CH2)_n-] \quad (I)$$

la masse moléculaire en poids dudit oligomère copolyester étant inférieure à 20 000, de préférence inférieure à 15 000.

La présente invention concerne également un procédé de traitement cosmétique de la peau, des muqueuses ou des phanères dans lequel on applique une composition selon l'invention sur la peau, les muqueuses ou les phanères.

## Description

La présente invention concerne une nouvelle composition topique comprenant un matériau gélifiant particulier.

Différents gélifiants ou épaississants usuels sont connus pour la préparation de compositions topiques, notamment les extraits d'algue tels que l'agaragar, les carraghénanes, les alginates; les gommes extraits de graines, exudats de plantes ou exudats de microorganismes, les dérivés cellulosiques; les extraits de fruits tels que les pectines; les agents gélifiants d'origine animale tels que la gélatine, les caséïnates ou les polymères synthétiques gélifiants hydrosolubles tels que les acides polyacryliques réticulés. De tels agents gélifiants permettent d'adapter la viscosité des compositions topiques en fonction de leur mode d'application et de l'effet recherché. Toutefois, ils présentent certains inconvénients, en particulier lorsqu'on augmente leur concentration dans les compositions, ils conduisent rapidement à des produits très visqueux difficiles à mettre en forme, diminuant en outre les propriétés d'étalement de ces compositions et leur facilité d'application sur la peau, les muqueuses ou les phanères, et diminuant en particulier leurs propriétés cosmétiques à l'application.

La Demanderesse a maintenant constaté qu'en employant un matériau gélifiant particulier, il était possible d'obtenir des compositions topiques présentant des formes diverses, liquides, pâteuses ou solides, tout en conservant une bonne facilité d'application topique.

La Demanderesse a également trouvé qu'en employant ce gélifiant particulier, il était possible d'obtenir un film après application et séchage d'une composition gélifiée le comprenant, ledit film et ses constituants ne transférant pas sur des supports avec lesquels ils peuvent entrer en contact (par exemple des tissus, des verres, des tasses, etc).

La présente invention concerne donc une composition topique comprenant une phase aqueuse laquelle comprend un agent gélifiant hydrophile particulier de type polyester sulphoné.

Les agents gélifiants particuliers utiles selon l'invention sont des oligomères copolyesters terephtaliques hydrosolubles ou hydrodispersables comprenant essentiellement des motifs répétitifs dicarboxylates de formule (I):

$$[-CO-A-CO-O-(CH_2-CH_2)_n-] \tag{I}$$

dans laquelle

A représente un groupement 1,4-phénylène, sulfo-1,3-phénylène et éventuellement 1,3-phénylène,
n va de 1 à 4,
au moins 35 % en mole desdits motifs de formule (I) étant des motifs de formule (I) pour lesquels A représente un groupement 1,4-phénylène et n est égal à 1,
au moins 7 % en mole desdits motifs de formule (I) étant des motifs de formule (I) pour lesquels A représente un groupement sulfo-1,3-phénylène,
la masse moléculaire en poids desdits oligomères copolyesters étant inférieure à 20 000, de préférence inférieure à 15 000.

De manière préférentielle, au moins 40 % en mole, plus préférentiellement entre 40 et 90 % en mole des motifs de formule (I) sont des motifs de formule (I) pour lesquels A représente un groupement 1,4-phénylène et n est égal à 1.

De préférence, au moins 10 % en mole, plus préférentiellement entre 10 % et 25 % en mole des motifs de formule (I) sont des motifs de formule (I) pour lesquels A représente un groupement sulfo-1,3-phénylène.

Les extrémités des chaînes desdits oligomères copolyesters peuvent être semblables ou différentes et représentées essentiellement par les groupements de formule (I'):

$$-CO-A-CO-O-(CH_2-CH_2)_n-OH \tag{I'}$$

dans laquelle A et n sont définis ci-dessus.

Lesdits oligomères peuvent également présenter en extrêmités de chaîne, et ce en quantités mineures des groupements de formules

$$-A-CO-OH$$

$$-A-CO-OR$$

formules dans lesquelles A est défini ci-dessus et R représente un groupement alkyle en $C_1$-$C_4$.

Lorsque A représente un groupement sulfo-1,3-phénylène, il s'agit plus particulièrement d'un sulfonate de métaux alcalins, notamment sodium ou potassium, ou d'un sulfonate d'ammonium ou de mono-, di-, tri- ou tétra- alkylammonium inférieur. Par alkylammonium inférieur, on entend de préférence selon l'invention un ammonium dont le ou les radicaux alkyles sont des alkyles inférieurs, de préférence en $C_1$-$C_6$. De manière préférentielle, il s'agit d'un sulfonate de sodium.

L'oligomère copolyester peut comprendre éventuellement jusqu'à 20 % en mole, de préférence jusqu'à 5 % en mole de motifs de formule (I) pour lesquels A représente un groupement 1,3-phénylène.

Selon un mode de réalisation préférentiel de l'invention, l'oligomère copolyester ci-dessus a une masse moléculaire en poids comprise entre 5 000 et 14 000, plus préférentiellement comprise entre 8 000 et 10 000.

Les masses moléculaires en poids sont mesurées par chromatographie par perméation de gel dans le diméthylacétamide contenant $10^{-2}$ N de LiBr, à 100°C. Les résultats sont exprimés en équivalents polystyrène.

Lesdits oligomères copolyesters peuvent être obtenus par les procédés usuels de préparation des polyesters par voie fondue, voie solvant ou voie interfaciale, procédés faisant intervenir des réactions

- . d'estérification de diacides et de diols et polycondensation
- . de transestérification de diesters et de diols et polycondensation
- . d'autocondensation d'hydroxyacides
- . de Schotten-Baumann par mise en oeuvre de diols et de chlorures d'acide et polycondensation
- . de polymérisation de lactones

en contrôlant la teneur minimum en motifs de formule (I) pour lesquels A représente un groupement 1,4-phénylène et n est égal à 1, semblables par les rapports stoechiométriques initiaux des différents monomères et par la maîtrise des réactions secondaires.

Un mode de préparation particulièrement intéressant est celui par transestérification / polycondensation et / ou d'estérification / polycondensation par voie fondue à l'aide d'un catalyseur de transestérification et/ou estérification.

La maîtrise de la structure est obtenue par contrôle de la teneur minimum en motifs de formule (I) pour lesquels A représente un groupement 1,4-phénylène et n est égal à 1, semblables par les rapports stoechiométriques initiaux des différents monomères diacides et/ou diesters et diol et par mise en oeuvre d'un agent limiteur d'éthérification, agent limiteur qui peut être un composé basique tel que les amines aliphatiques ou aromatiques ou un hydroxyde ou acétate de métaux alcalins ou alcalino-terreux.

Le contrôle de la masse moléculaire est obtenu d'une manière connue de l'homme du métier, par compromis adéquat entre la pression, la température et le temps.

Les nouveaux oligomères copolyesters téréphtaliques faisant l'objet de l'invention peuvent être préparés par estérification et/ou transestérification / polycondensation d'une composition monomère à base:

- d'acide, anhydride ou diester téréphtalique (Tp)
- d'acide, anhydride ou diester sulfoisophtalique (SIp)
- éventuellement d'acide, anhydride ou diester isophtalique (Ip)
- et d'éthylène glycol (EG)

selon des quantités relatives correspondant à

* un rapport molaire (SIp) / [(Tp)+(SIp)+(Ip)] d'au moins 7/100, de préférence d'au moins 10/100, tout particulièrement de 10/100 à 25/100
* un rapport molaire (Ip) / [(Tp)+(SIp)+(Ip)] de 20/100 au plus, de préférence de 5/100 au plus
* un rapport molaire (EG) / [(Tp)+(SIp)+(Ip)] de 2/1 à 3/1.

en présence d'un catalyseur d'estérification et/ou transestérification et d'un limiteur de d'éthérification.

Le monomère téréphtalique (Tp) est de préférence mis en oeuvre sous la forme de diester inférieur (diester de dialkyle en $C_1$-$C_4$), de diméthyle de préférence.

Le monomère sulfoisophtalique (SIp) est de préférence mis en oeuvre sous la forme d'un sulfonate de métal alcalin (sodium notamment) de diester inférieur (d'alkyle en $C_1$-$C_4$), de méthyle de préférence. On peut citer tout particulièrement le sodio-oxysulfonyl-5 isophtalate de diméthyle.

Le monomère isophtalique (Ip) éventuel est de préférence mis en oeuvre sous la forme d'acide isophtalique.

Lorsque tous les monomères "diacides" sont mis en oeuvre sous la forme d'un diesters, l'opération de transestérification (interéchange) entre ces monomères "diacides" et l'éthylène glycol est effectuée à une température supérieure ou égale à 130°C, de préférence de l'ordre 140 à 220°C et tout particulièrement de l'ordre de 180 à 220°C ; à cette température le méthanol (cas préférentiel des diesters méthyliques) formé est éliminé du milieu réactionnel de préférence par distillation.

Cette opération d'interéchange est réalisée en présence d'un catalyseur de transestérification métallique et d'un limiteur d'éthérification. Ledit catalyseur est de préférence un carboxylate métallique, tel que l'acétate de manganèse, l'acétate de zinc, l'acétate de cobalt ou l'acétate de calcium, ou d'un titanate organique ou minéral, tel que le titanate de butyle, le titanate de nitrilo-2,2',2"-triéthyle (ou aminotriéthanolate de titane jouant en outre le rôle de limiteur d'éthérification) ou le titanate de calcium. Les catalyseurs préférés sont les titanates organiques ; ils sont mis en oeuvre en quantités de l'ordre d'au moins 0,001% en poids exprimé en titane, de préférence de l'ordre de 0,002% à 0,02% en poids de titane par rapport au poids de réactifs présents.

L'agent limiteur d'éthérification peut être un composé basique tel que les amines aliphatiques ou aromatiques (triéthanolamine, carbonate de guanidine, diméthylaniline, naphtylamine ...) ou un hydroxyde ou acétate de métaux alcalins ou alcalino-terreux (acétate de sodium, potassium, benzoate de sodium ...). Il est mis en oeuvre généralement en quantité de l'ordre de 0,001% à 0,05% par rapport au poids de réactifs présents.

La durée de l'opération d'interéchange est de 1 à 4 heures ; elle est généralement de l'ordre de 2 à 3 heures.

Lorsque plus de 90% de la quantité théorique de méthanol a été distillée, le polyol excédentaire est éliminé en portant la température du milieu réactionnel à 230°C.

L'opération de polycondensation est de préférence réalisée à une température de l'ordre de 230 à 280°C, de préférence de l'ordre de 240 à 260°C, dans un autre réacteur préalablement porté à cette température et progressivement mis sous vide jusqu'à une pression qui peut aller jusqu'à 10 Pa ; une réduction de pression jusqu'à 10 millibar environ dure de l'ordre de 40 minutes.

L'opération de polycondensation se déroule avec élimination de molécules de polyol, cette opération est stoppée lorsque le couple moteur de l'arbre d'agitation indique une valeur équivalente à environ 0,5 à 5 mètres.newton pour une température de 250°C de la masse réactionnelle et une vitesse d'agitation de 80 tours / minute d'un mobile en forme d'ancre dans un réacteur de 7,5 litres. Le vide est ensuite cassé à l'azote, et le polymère est coulé dans une lingotière ; après refroidissement, le polymère est broyé.

Lorsque l'un des monomères "diacides" est présent sous forme diacide ou anhydride et le ou les autres sous forme de diester(s), lesdits oligomères copolyesters sont obtenus en réalisant d'abord une opération de transestérification des monomères diesters avec de l'éthylène glycol dans les conditions ci-dessus décrites, suivie d'une opération d'estérification dans le milieu du monomère diacide ou anhydride avec de l'éthylène glycol, puis polycondensation dans les conditions décrites ci-dessus, la quantité totale d'éthylène glycol étant répartie entre les deux opérations (transestérification et estérification).

Si nécessaire, l'opération d'estérification est réalisée par ajout dans le milieu réactionnel résultant de l'opération de transestérification, du monomère sous forme diacide ou anhydride et d'éthylène glycol préalablement mis en suspension, à une température correspondant à celle de la fin de la température d'interéchange ; la période d'introduction est de l'ordre de 1 heure.

Cette opération d'estérification est réalisée à une température de l'ordre de 230 à 280°C, de préférence de l'ordre de 250 à 260°C, en présence d'un catalyseur du même type que celui de transestérification et d'un agent limiteur d'éthérification.

L'opération est réalisée en présence des mêmes types de catalyseur et de limiteur d'éthérification que ceux mis en oeuvre lors de l'opération de transestérifiacation, et ce dans les mêmes proportions.

La réaction s'effectue avec élimination d'eau qui est soutirée du réacteur en même temps que le polyol en excès.

Ce type de procédé de préparation est notamment décrit dans la demande de brevet WO 95/32997 (RHONE-POULENC CHIMIE).

De manière préférentielle, la composition selon l'invention comprend entre 0,001 et 40 % en poids par rapport au poids total de la composition d'oligomère copolyester terephtalique. Selon la forme recherchée, liquide, pâteuse ou solide, on emploiera des quantités différentes de gélifiant particulier. Pour une composition solide, on emploiera de préférence entre 10 et 40 % en poids de gélifiant particulier, plus préférentiellement entre 20 et 30 % en poids. Pour une composition pâteuse, on emploiera de préférence entre 0,5 et 10 % en poids de gélifiant particulier, plus préférentiellement entre 2 et 5 % en poids. Des quantités inférieures seront employées pour une composition de consistance liquide.

La composition selon l'invention est de manière préférentielle une composition cosmétique ou pharmaceutique, destinée à être appliquée sur la peau, les muqueuses ou les phanères.

Elle peut être employée pour toutes les utilisations dermo-cosmétiques usuelles, et notamment comme composition d'hygiène corporelle telle qu'un stick déodorant; comme composition capillaire telle qu'un gel de coiffage; comme composition de maquillage, notamment comme composition de maquillage dit « sans transfert », en particulier un rouge à lèvres ; comme composition de soin, par exemple une lotion hydratante ; ou encore comme produit de soin bucco-dentaire tel que des pâtes ou des gels dentifrices, des bains de bouche.

La composition selon l'invention peut donc comprendre d'autres constituants usuels en cosmétique selon l'utilisation qui est envisagée.

La composition selon l'invention peut comprendre en outre une phase grasse. La phase grasse peut comprendre des huiles ou cires usuelles en cosmétique, d'origine animale, végétale, minérale ou synthétique, seules ou en mélanges.

La composition selon l'invention peut comprendre des additifs et/ou des actifs usuels en dermo-cosmétique, étant entendu que l'homme du métier saura déterminer les quantités de ces additifs et actifs susceptibles d'être ajoutés à la composition selon l'invention de manière à ne pas altérer les propriétés de l'agent gélifiant particulier selon l'invention.

Les additifs usuels en cosmétique sont en particulier des parfums, des colorants, des absorbeurs d'odeur, des additifs assurant la stabilité de la composition comme des conservateurs, des filtres U.V.A et/ou U.V.B., des antioxydants hydrophiles et/ou lipophiles, des chélatants, etc. Les quantités de ces différents adjuvants sont celles classiquement utilisées dans le domaine considéré, et par exemple de 0,0001 à 5 % en poids du poids total de la composition. Ces adjuvants, selon leur nature, peuvent être introduits dans la phase aqueuse ou dans la phase grasse lorsque la composition comprend également une phase grasse.

La composition selon l'invention peut également comprendre des actifs usuels en cosmétique, hydrophiles et/ou lipophiles, en particulier des agents anti-radicaux libres, des alpha- ou bêta-hydroxyacides, des filtres U.V.A et/ou U.V.B., des céramides, des agents antipelliculaires comme l'octopirox ou la pyrithione de zinc, des agents antiacnéiques comme l'acide rétinoïque ou le peroxyde de benzoyle, des agents anti-chute des cheveux comme le minoxidil, des agents antifongiques ou antiseptiques, des agents pour le soin bucco-dentaire etc.

Pour une utilisation comme composition d'hygiène corporelle, la composition selon l'invention peut comprendre des produits déodorants contenant des substances actives de type antitranspirant et/ou de type bactéricide pour diminuer voire supprimer les odeurs axillaires généralement désagréables, et/ou des absorbeurs d'odeurs.

Pour une utilisation comme composition capillaire, la composition selon l'invention, du fait des propriétés rhéologiques spécifiques du gel la constituant, apporte un bon effet coiffant et de la discipline dans la coiffure.

Pour obtenir un effet fixant ou améliorer l'effet coiffant et démêlant, on peut ajouter à la composition selon l'invention un matériau fixant ou un matériau conditionneur. Ces matériaux fixants ou conditionneurs peuvent être employés dans des quantités comprises entre 0,01 et 15 % en poids par rapport au poids total de la composition, de préférence entre 0,1 et 8 % en poids.

La composition selon l'invention peut également comprendre des actifs pour le soin des cheveux et/ou des agents de renfort de brillance et/ou des agents de coloration capillaire. Ces actifs et/ou agents capillaires peuvent être employés dans des quantités comprises entre 0,01 et 20 % en poids par rapport au poids total de la composition selon l'invention.

Pour une utilisation comme composition de maquillage, la composition selon l'invention peut comprendre des matériaux usuels pour les compositions de maquillage, en particulier des charges et/ou des matériaux colorants.

Lorsque la composition est une composition de soin, elle comprend des actifs comme l'acide ascorbique, l'acide kojique, l'acide citrique, l'acide caféique, l'acide salicylique et ses dérivés (par exemple acide n-octanoyl-5 ou décanoyl-5-salicylique), les $\alpha$-hydroxyacides, l'acide rétinoïque et ses dérivés tels que le rétinol et les esters de rétinol, l'acide benzène 1,4-di(3-méthylidène 10-camphosulfonique). On peut aussi, utiliser tous les composés naturels ou synthétiques contenant de tels acides, comme les extraits végétaux et plus spécialement les extraits de fruits. On peut aussi solubiliser les dérivés xanthiques (caféine, théophylline), l'acide $\beta$-glycyrrhétinique ou l'acide asiatique.

En fonction de l'utilisation envisagée et des propriétés physicochimiques et/ou cosmétiques de la composition selon l'invention, elle peut également comprendre des gélifiants additionnels de manière à modifier ses propriétés de douceur ou de dureté, elle peut comprendre également des latex ou pseudolatex qui augmentent la rémanence à l'eau de la composition et lui apportent de la brillance, ou encore une ou plusieurs gommes de silicone qui permettent de conférer aux compositions finales des qualités de douceur et de glissant.

L'homme du métier saura déterminer le procédé de préparation de la composition selon l'invention en fonction de ses constituants.

La présente invention concerne également un procédé de traitement cosmétique de la peau, des muqueuses, des cheveux ou des phanères dans lequel on applique la composition telle que définie ci-dessus sur la peau, les muqueuses ou les phanères.

Les exemples ci-après permettent d'illustrer la présente invention sans toutefois chercher à en limiter la portée. Les pourcentages sont exprimés en poids par rapport au poids total des composition. Les pourcentages de copolymère isophtalate / téréphtalate / sulfoisophtalate d'éthylène glycol dans les compositions sont des pourcentages de matière active.

### Exemple 1 Préparation d'un oligomère copolyester téréphtalique selon l'invention

Dans un réacteur en acier inoxydable de 7,5 litres, muni d'un agitateur à ancre tournant à 80 tr/mn relié à un couplemètre KYOWA, d'une double enveloppe pour la circulation d'un liquide caloporteur et d'une colonne à distiller régulée par une électrovanne on introduit :

- 11,47 moles de téréphtalate de diméthyle
- 2,53 moles de diméthyl-5 sulfonate de sodium
- 39,16 moles d'éthylène glycol
- 54 ppm en poids de titane, sous forme d'aminotriéthanolate de titane comme catalyseur et agent limiteur d'éthérification.

Le mélange est préchauffé à 180°C. Il est ensuite porté jusqu'à la température de 220°C en environ 130 minutes, pour distiller plus de 90% de la quantité théorique de méthanol.

Le mélange réactionnel est ensuite amené à 230°C en 30 minutes. Lorsque la masse réactionnelle a atteint cette température, on introduit en 60 minutes, toujours à 230°C, une suspension dont la composition est la suivante :

- 0,5 mole d'acide isophtalique
- 2,36 moles d'acide téréphtalique
- 8 moles d'éthylène glycol

La masse réactionnelle est alors amenée à la température de 250°C en 60 minutes.

Pendant la période d'introduction du mélange et pendant la période de chauffage jusqu'à 250°C, on distille un mélange d'eau et d'éthylène glycol sans rétrogradation.

Le mélange réactionnel est ensuite transféré dans un autoclave préchauffé à 250°C puis mis sous pression réduite de 100 millibar en 22 minutes. Après 2 minutes dans ces conditions de température et de pression, la masse réactionnelle est coulée et refroidie.

Le copolyester obtenu présente les caractéristiques structurales décrites au tableau 1.

### Exemple 2 Préparation d'un oligomère copolyester téréphtalique hors l'invention

Dans un réacteur en acier inoxydable identique à celui décrit dans l'exemple 1 on introduit les réactifs suivants :

- 15,16 moles de téréphtalate de diméthyle
- 1,99 mole de diméthyl-5 sulfonate de sodium
- 48 moles d'éthylène glycol
- 54 ppm en poids de titane, sous forme d'orthotitanate de butyle comme catalyseur et agent limiteur d'éthérification.

Le mélange est préchauffé à 180°C. Il est ensuite porté jusqu'à la température de 220°C en environ 130 minutes, pour distiller plus de 90% de la quantité théorique de méthanol.

Le mélange réactionnel est ensuite amené à 250°C en 90 minutes. Lorsque la masse réactionnelle a atteint cette température, le mélange réactionnel est transféré dans un autoclave préchauffé à 250°C puis mis sous pression réduite de 1 millibar en 60 minutes. Le mélange réactionnel est ensuite maintenu dans ces conditions de température et de pression pendant 90 minutes à la suite desquelles, la masse réactionnelle est coulée et refroidie.

Le copolyester obtenu présente les caractéristiques structurales décrites au tableau 1.

Dans ce tableau:

* "% molaire des motifs diacides" correspond à la teneur, en %, de chaque diacide ou diester mise en oeuvre par rapport à l'ensemble des diacides ou diesters mis en oeuvre.

"Tp" signifie : motif téréphtalique
"Ip" signifie : motif isophtalique
"SIp" signifie : motif sulfoisophtalique

* Les caractéristiques de la partie "glycol" des copolyesters sont obtenues par méthanolyse des produits à 190°C pendant 16 heures suivies d'une analyse par la technique de chromatographie en phase vapeur et dosage par étalonnage interne.

- "% molaire des motifs diols" correspond à la teneur, en %, des motifs oxyéthylène "G", des motifs di(oxyéthylène) "2G", des motifs tri(oxyéthylène) "3G" et des motifs tetra(oxyéthylène) "4G", par rapport à l'ensemble des motifs diols.

\* "%GT/Σ motifs" correspond au % molaire des motifs de formule (I)

$$[-CO-A-CO-O-(CH_2-CH_2-O)_n-] \tag{I}$$

où A est 1,4 phénylène et n=1
par rapport à l'ensemble des motifs de formule (I)
où A est 1,4 phénylène, sulfo1,3 phénylène et éventuellement 1,3 phénylène et n va de 1 à 4
"%GT/Σ motifs" se calcule par la formule suivante :

$$\%GT/\Sigma \text{ motifs} = (\% \text{ molaire de motifs Tp}) \times (\% \text{ molaire de motifs G})/100$$

\* La masse molaire des polyesters (Mw) est déterminée par chromatographie par permation de gel (GPC) dans le DMAc/LiBr à 100%, les résultats sont données en équivalents polystyrène.

| exemple | 1 | 2 (comparatif) |
|---|---|---|
| % molaire des motifs diacides | | |
| Tp | 82 | 88,4 |
| Ip | 3 | 0 |
| SIp | 15 | 11,6 |
| %GT/Σ motifs | 46,5 | 47 |
| % molaire des motifs diols | | |
| G | 56,8 | 52,8 |
| 2G | 30,7 | 34,2 |
| 3G | 10 | 10,7 |
| 4G | 2,5 | 2,3 |
| Mw | 8 000 | 50 000 |

### Exemple 3 Gel à 20 % en oligomère

On prépare un gel aqueux en mélangeant à froid 20 % d'oligomère de l'exemple 1 et le complément à 100 % d'eau déminéralisée. Le gel fluide obtenu est coulé dans un moule et laissé reposer 24 heures. Après prise en masse, on obtient un stick de coiffage de viscosité initiale égale à 90000 Pa.s. Sous une contrainte de cisaillement de 1500 Pa, la viscosité est de 30 Pa.s.

De telles caractéristiques rhéologiques permettent l'application de films à partir de la composition topique solide selon l'invention, sur des surfaces molles ou mouvantes comme les cheveux.

En revanche, avec le polymère de l'exemple 2 (comparatif), qui se différencie essentiellement des oligomères selon l'invention par une masse moléculaire en poids de 50 000, la composition aqueuse à 20 % en poids reste liquide, même après plus de 24 heures au repos.

### Exemple 4 Gel à 8 % en oligomère

On prépare un gel aqueux en mélangeant à froid 8 % d'oligomère de l'exemple 1 et le complément à 100 % d'eau déminéralisée. Le gel fluide obtenu est coulé dans un moule et laissé reposer 24 heures. Après prise en masse, on mesure le gel obtenu présente une viscosité initiale de 30 000 Pa.s, et une viscosité sous contrainte de cisaillement à 105 Pa de30 Pa.s.

### Exemple 5 Gel conditionneur

On reprend le mode opératoire de l'exemple 3 avec les constituants suivants:

| | |
|---|---|
| Oligomère de l'exemple 1 | 5 % ma |

(suite)

| Polyaminosiloxane (commercialiée sous la dénomination DC 939 par la société DOW CORNING) | 2 % ma |
|---|---|
| Eau        qsp | 100 % |

## Revendications

**1.** Composition topique comprenant une phase aqueuse laquelle comprend un agent gélifiant hydrophile, caractérisée en ce que le gélifiant hydrophile de type polyester sulphoné est un oligomère copolyester terephtalique hydrosoluble ou hydrodispersable comprenant essentiellement des motifs répétitifs dicarboxylates de formule (I):

$$[-CO-A-CO-O-(CH_2-CH_2)_n-] \tag{I}$$

dans laquelle

A représente un groupement 1,4-phénylène, sulfo-1,3-phénylène et éventuellement 1,3-phénylène,
n va de 1 à 4,
au moins 35 % en mole, préférentiellement au moins 40 % en mole, plus préférentiellement entre 40 et 90 % en mole desdits motifs de formule (I) étant des motifs de formule (I) pour lesquels A représente un groupement 1,4-phénylène et n est égal à 1,
au moins 7 % en mole, préférentiellement au moins 10 % en mole, plus préférentiellement entre 10 % et 25 % en mole desdits motifs de formule (I) étant des motifs de formule (I) pour lesquels A représente un groupement sulfo-1,3-phénylène,
l'oligomère copolyester pouvant comprendre éventuellement jusqu'à 20 % en mole, de préférence jusqu'à 5 % en mole de motifs de formule (I) pour lesquels A représente un groupement 1,3-phénylène, et
la masse moléculaire en poids dudit oligomère copolyester étant inférieure à 20 000, de préférence inférieure à 15 000.

**2.** Composition selon la revendication 1, caractérisée en ce que l'oligomère copolyester a une masse moléculaire en poids comprise entre 5 000 et 14 000, plus préférentiellement comprise entre 8 000 et 10 000.

**3.** Composition selon l'une des revendications 1 ou 2, caractérisée en ce qu'elle comprend entre 0,001 et 40 % en poids par rapport au poids total de la composition d'oligomère copolyester terephtalique.

**4.** Composition selon la revendication 3, caractérisée en ce qu'elle comprend entre 10 et 40 % en poids d'oligomère copolyester terephtalique, préférentiellement entre 20 et 30 % en poids.

**5.** Composition selon la revendication 3, caractérisée en ce qu'elle comprend entre 0,5 et 10 % en poids d'oligomère copolyester terephtalique, préférentiellement entre 2 et 5 % en poids.

**6.** Composition selon l'une des revendications 1 à 5, caractérisée en ce qu'il s'agit d'une composition cosmétique ou pharmaceutique.

**7.** Composition selon l'une des revendications 1 à 6, caractérisée en ce qu'il s'agit d'une composition d'hygiène corporelle, d'une composition capillaire, d'une composition de maquillage ou d'une composition de soin.

**8.** Composition selon l'une des revendications 1 à 7, caractérisée en ce qu'elle contient des additifs et/ou des actifs usuels en dermo-cosmétique choisis dans le groupe constitué par des parfums ; des stabilisants comme des conservateurs ; des filtres U.V.A et/ou U.V.B. ; des antioxydants hydrophiles et/ou lipophiles ; des chélatants ; des actifs hydrophiles et/ou lipophiles, en particulier des agents anti-radicaux libres, des alpha- ou bêta-hydroxyacides, des céramides ; des agents antipelliculaires, des agents antiacnéiques, des agents anti-chute des cheveux, des agents antifongiques ou antiseptiques, , des agents pour le soin bucco-dentaire, des substances actives de type antitranspirant et/ou de type bactéricide et/ou absorbeurs d'odeurs ; des matériaux fixants ou des matériaux conditionneurs des cheveux; des actifs pour le soin des cheveux et/ou des agents de renfort de brillance et/ou des agents de coloration capillaire ; des charges ; des matériaux colorants ; des gélifiants ; des gommes de silicone.

9. Composition selon l'une des revendications 1 à 8, caractérisée en ce qu'elle contient en plus au moins un latex ou un pseudolatex.

10. Composition selon l'une des revendications 7 à 9, caractérisé en ce qu'il s'agit d'une composition de maquillage dit « sans transfert ».

11. Composition selon la revendication 7 à 10, caractérisée en ce qu'il s'agit d'un rouge à lèvres.

12. Composition selon l'une des revendications 7 à 9, caractérisée en ce qu'il s'agit d'un produit de soin bucco-dentaire.

13. Composition selon la revendication 12, caractérisée en ce qu'il s'agit d'un gel ou d'une pâte dentifrice ou bien d'un bain de bouche.

14. Procédé de traitement cosmétique de la peau, des muqueuses ou des phanères dans lequel on applique une composition selon l'une des revendications 1 à 13 sur la peau, les muqueuses ou les phanères.

**Office européen des brevets** | **RAPPORT DE RECHERCHE EUROPEENNE** | Numéro de la demande

EP 98 40 0398

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | US 4 300 580 A (O'NEILL GEORGE J ET AL) 17 novembre 1981 <br> * le document en entier * <br> --- | 1-8,10, 14 | A61K7/48 <br> A61K7/06 <br> A61K7/32 <br> A61K7/02 |
| X | WO 96 03964 A (PROCTER & GAMBLE ;LANGLOIS ANNE (GB)) 15 février 1996 <br> * le document en entier * <br> --- | 1-8,10, 14 | |
| X | US 4 525 524 A (TUNG WILLIAM C T ET AL) 25 juin 1985 <br> * page 1-7; revendications 1-11 * <br> --- | 1-8,10, 14 | |
| X | CHEMICAL ABSTRACTS, vol. 120, no. 14, 4 avril 1994 Columbus, Ohio, US; abstract no. 173134, JUCHI, MINAKO ET AL: "Cosmetics containing polyester particles" XP002053931 <br> * abrégé * <br> & JP 05 310 530 A (TOYO BOSEKI, JAPAN) <br> ----- | 1-8,10, 14 | |

| | | DOMAINES TECHNIQUES RECHERCHES (Int.Cl.6) |
|---|---|---|
| | | A61K |

Le présent rapport a été établi pour toutes les revendications

| Lieu de la recherche | Date d'achèvement de la recherche | Examinateur |
|---|---|---|
| LA HAYE | 22 juin 1998 | Sierra Gonzalez, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)